# FASCICULE DE BREVET EUROPEEN

(11) **EP 3 125 867 B1**
(45) Date de publication et mention de la délivrance du brevet: **15.09.2021**
(21) Numéro de dépôt: 15723135.8
(22) Date de dépôt: 01.04.2015
(51) Int. Cl.: A61K 9/00, A61K 31/728, A61P 27/02

(54) **COMPOSITION HYPEROSMOLAIRE D'ACIDE HYALURONIQUE**
HYPEROSMOLARE ZUSAMMENSETZUNG VON HYALURONSÄURE
HYPEROSMOLAR COMPOSITION OF HYALURONIC ACID

(30) Priorité: 02.04.2014 FR 1452929
(43) Date de publication de la demande: 08.02.2017
(73) Titulaire: Horus Pharma, 06700 Saint-Laurent-du-Var (FR)
(72) Inventeur: ROULAND, Jean-François, F-59000 Lille (FR); CLARET, Martine, CH-1025 Saint Sulpice (CH); CLARET, Claude, CH-1025 Saint Sulpice (CH)
(74) Mandataire: Be IP
(86) Numéro de dépôt international: PCT/EP2015/057186
(87) Numéro de publication internationale: WO 2015/150459

(56) Documents cités:
- EP-A1- 0 323 522
- WO-A1-2012/013736
- WO-A1-2013/043832
- KNEZOVIC IGOR ET AL: "Therapeutic efficacy of 5% NaCl hypertonic solution in patients with bullous keratopathy.", COLLEGIUM ANTROPOLOGICUM JUN 2006, vol. 30, no. 2, June 2006 (2006-06), pages 405-408, XP009195268, ISSN: 0350-6134

## Description

La présente invention concerne une nouvelle composition hyperosmolaire d'acide hyaluronique pour son utilisation dans le traitement de l'œdème cornéen.

L'œdème cornéen résulte d'une infiltration de liquide dans les couches de la cornée. Il se manifeste par un épaississement de la cornée avec perte de sa transparence et baisse de l'acuité visuelle du sujet atteint par cette affection. Les traitements usuels de l'œdème cornéen se font par application topique de collyres anti-inflammatoires et antiœdémateux, notamment des solutions hypertoniques de chlorure de sodium. Ces traitements qui permettent de soulager de manière temporaire les sujets atteints d'œdème cornéen, se révèlent toutefois peu efficaces dans le cas d'œdèmes chroniques puisqu'ils ne permettent pas de prévenir de nouvelles infiltrations de liquide.

Dans les cas les plus graves, il est nécessaire de faire appel à des traitements chirurgicaux comme une greffe de cornée ou des traitements par laser. Il est donc important de pouvoir traiter de manière efficace ces œdèmes de manière à éviter les récidives et complications nécessitant à terme le recours à ces traitements chirurgicaux.

On connaît de l'état de la technique des compositions salines comprenant de l'acide hyaluronique. Il s'agit toutefois de « larmes artificielles » isotoniques voire très légèrement hypertoniques, mais très proches de l'osmolarité des larmes (isotonicité). Ainsi, EP 323 522 décrit plusieurs exemples de compositions identifiées comme larmes artificielles comprenant de l'acide hyaluronique et du chlorure de sodium, avec un rapport d'osmolarité par rapport à une solution saline isotonique de 1 à 1,1. Ces larmes artificielles sont employées pour traiter la sécheresse oculaire. WO 2013/043832 décrit également une composition ophtalmique pour le traitement de l'érosion cornéenne récurrente (RCE) comprenant, notamment, de l'acide hyaluronique et du chlorure de sodium. Cette composition est présentée comme hypertonique, mais avec une pression osmotique comprise entre 320 à 350 mOsmol/kg, soit une pression très proche s'une solution saline isotonique du même ordre que celle des exemples de formulations de la demande EP 323 522. Aucune de ces solutions salines d'acide hyaluronique n'a une osmolarité suffisante pour le traitement de l'œdème cornéen, personne n'envisageant même d'employer de l'acide hyaluronique dans une solution hyperosmolaire pour traiter l'œdème cornéen.

Ainsi, Knezovic & al (Collegium Antropologicum, vol. 30, n° 2, juin 2006, 405-408) décrit une composition saline hyperosmolaire de 5% de NaCl pour le traitement de la keratopathie bulleuse, sans usage d'acide hyaluronique. De même, les formulations commerciales comme le Muro 128 ne comprennent pas d'acides hyalmuronique.

US 2010/068300 décrit une composition comprenant de la L-carnitine (10%) et de la taurine (2%), pour le traitement de patients ayant des œdèmes cornéens dus à une dystrophie de Fuschs. La composition comprend également 0,2% d'acide hyaluronique comme agent humidifiant avec une osmolarité d'au plus 1200 mOsm/L.

### BRÈVE DESCRIPTION DE L'INVENTION

La présente invention concerne une composition ophtalmique hyperosmolaire comprenant de l'acide hyaluronique ou un sel acceptable de ce dernier et un agent d'hyperosmolarité, la composition ayant une osmolarité allant de 1500 à 2000 mOsm/l et une une viscosité allant de 15 à 100 mPa.s (15 à 100 centipoises). Cette viscosité est mesurée selon les préconisations de la Pharmacopée Européenne 2.2.10, avec un viscosimètre rotatif, à 25°C, et 100s⁻¹.

L'invention concerne également une composition ophtalmique hyperosmolaire comprenant de l'acide hyaluronique ou un sel acceptable de ce dernier et un agent d'hyperosmolarité pour son utilisation dans le traitement de l'œdème cornéen, et plus particulièrement pour le traitement de l'œdème cornéen chronique (OCC).

### DESCRIPTION DÉTAILLEE DE L'INVENTION

L'objet de la présente invention est une composition ophtalmique, c'est à dire une composition destinée à être appliquée sur l'œil d'un sujet, humain ou animal, plus particulièrement humain. En conséquence, la composition ophtalmique doit répondre à des caractéristiques techniques spécifiques des compositions ophtalmiques, et plus particulièrement liées à la sélection de ses composants. Ces composants qui sont « ophtalmiquement acceptables » ne doivent pas, individuellement ou associés dans la composition, provoquer de réactions secondaires de l'œil en dehors de l'effet recherché par la composition et ses actifs. L'œil étant un organe particulièrement sensible à un stress environnemental, la composition ne doit pas provoquer d'irritations parasites ou de réactions de type allergiques au détriment recherché, plus particulièrement dans le cas de compositions ophtalmiques destinées à traiter une affection ophtalmique. Le choix des constituants de la composition est donc très important, qui distingue la composition ophtalmique d'une simple composition inadaptée pour un usage ophtalmique. L'homme du métier est à même de choisir lesdits composants et de différencier une composition ophtalmique d'une simple composition destinée à un autre usage.

La composition ophtalmique a de préférence un pH compris entre 6 et 7. Elle comprend donc généralement un tampon approprié pour un usage ophtalmique, connu de l'homme du métier. On citera en particulier le citrate trisodique dihydraté et l'acide citrique monohydraté employés seuls ou en mélange.

La composition ophtalmique doit également être stérile pour ne pas apporter de pathogènes susceptibles de se développer et entrainer des complications ophtalmiques. Par « stérile », on entend au sens de la présente invention l'absence de germes au sens de la Pharmacopée Européenne, 8ème édition (2014).

De nombreuses compositions ophtalmiques comprennent des agents conservateurs pour éviter leur contamination par des germes, comme les ammoniums quaternaires, notamment le chlorure de benzalkonium, l'alkyl-diméthyl-benzylammonium, le cétrimide, le chlorure de cétylpyridinium, le bromure de benzododécinium, le chlorure de benzothonium,le chlorure de cetalkonium, les conservateurs mercuriels, comme le nitrate/acétate/borate phénylmercurique, le thiomersal, les conservateurs alcooliques, comme le chlorobutanol, l'alcool benzylique, le phényléthanol, l'alcool phénylethylique, les acides carboxyliques, tels que l'acide sorbique, les phénols, en particulier méthyl/propyl parabène, les amidines, par exemple le chlorhexidine digluconate et/ou des agents chélateur comme l'EDTA seul ou en association avec au moins un autre conservateur.

La composition selon l'invention est substantiellement dépourvue de tels conservateurs pour répondre à une indication « sans conservateurs ». Sa teneur en conservateurs est inférieure ou égale à 10 ppm, plus particulièrement inférieure ou égale à 1 ppm, préférentiellement égale à 0 ppm, aucun conservateur n'entrant dans sa composition.

En l'absence de conservateurs, la composition ophtalmique doit subir un traitement particulier au cours de sa préparation de manière à éviter et prévenir la contamination par des pathogènes. En ce sens, une composition ophtalmique dépourvue de conservateurs se distingue d'une simple composition comprenant de l'acide hyaluronique obtenue sans montrer de précautions particulières ni décrivant d'étapes du procédé permettant d'obtenir cette stérilité caractéristique des compositions ophtalmiques.

Les compositions ophtalmiques se présentent généralement sous forme de solutions, mais aussi sous forme de gels ou de pommades. La composition selon l'invention est de préférence une solution pour une application par instillation d'une ou de plusieurs gouttes dans l'œil. La viscosité de la solution est néanmoins choisie de manière à permettre son maintien sur l'œil, en particulier sur la cornée pendant un temps suffisant pour lui permettre d'agir.

La composition ophtalmique selon l'invention a de préférence une viscosité allant de 15 à 100 mPa.s (15 à 100 centipoises). Cette viscosité est mesurée selon les préconisations de la Pharmacopée Européenne 2.2.10, avec un viscosimètre rotatif, à 25°C, et 100s⁻¹. D'autres appareils de mesure et méthodes adaptés à la mesure de la viscosité de solutions sont connus de l'homme du métier et permettent d'obtenir des résultats similaires.

La viscosité de la composition ophtalmique selon l'invention tient d'abord à la quantité d'acide hyaluronique présente. Elle est ensuite adaptée par l'ajout d'agents de viscosités « ophtalmiquement acceptables ». L'homme du métier connaît bien les agents de viscosité susceptibles d'être employés pour la préparation de compositions ophtalmiques et les quantités à employer pour obtenir la viscosité recherchée. On citera en particulier l'hydroxypropylméthylcellulose, l'hydroxyethylcellulose, la carboxymethylcellulose, carbomères, les gels agar, polyvinylpyrrolidone et l'alcool polyvinylique.

De manière préférentielle, la composition ophtalmique selon l'invention comprend de l'hydroxypropylméthylcellulose, préférentiellement à une teneur allant de 0,05 à 0,5 % en poids d'hydroxpropylmethylcellulose, avantageusement de 0,1 à 0,4 % en poids, plus avantageusement de 0,2 à 0,3 % en poids, particulièrement environ 0,25 % en poids.

Sauf indication contraire, les pourcentages sont exprimés en poids par rapport au poids total de la composition.

La composition selon l'invention est une composition hyperosmolaire. Cette hyperosmolarité est définie par rapport à une valeur standard qui est celle du sérum physiologique représentatif du liquide lacrymal. Une composition « hyperosmolaire » est également appelée « hypertonique ». Le sérum physiologique a une valeur d'osmolarité de 300 mOsm/l mesurée avec un osmomètre selon la méthode décrite au paragraphe 2.2.35 de la Pharmacopée Européenne. La composition ophtalmique selon l'invention présente une osmolarité allant de 1500 à 2000 mOsm/l. De manière préférentielle, la composition a une osmolarité supérieure à 1500 mOsm/l, plus préférentiellement entre 1600 et 1900 mOsm/l, avantageusement environ 1750 mOsm/l.

L'homme du métier connaît bien les agents d'osmolarité d'usage ophtalmique. On citera en particulier des sels de sodium ou de potassium comme le chlorure de sodium et le chlorure de potassium et des polyols comme le glycérol. Ces agents d'osmolarité peuvent être employés seuls ou en mélange, comme par exemple un mélange de sels de sodium et de potassium, par exemple NaCl + KCl, ou comme un mélange d'un ou de plusieurs sels et d'un ou plusieurs polyols, comme des mélanges NaCl + glycérol, KCl + glycérol ou encore NaCl + KCl + glycérol. L'homme du métier sait comment employer ces agents d'osmolarité, les quantités à employer pour obtenir le rapport d'osmolarité recherché dans la composition ophtalmique selon l'invention, notamment en fonction également de la viscosité recherchée.

Selon un mode préférentiel de réalisation de l'invention, l'agent d'osmolarité est le chlorure de sodium. La composition ophtalmique comprend avantageusement de 2 à 10 % en poids de chlorure de sodium, avantageusement de 4 à 6 % en poids, plus avantageusement environ 5% en poids.

La composition ophtalmique selon l'invention comprend de l'acide hyaluronique ou un sel physiologiquement acceptable de ce dernier. De manière avantageuse, l'acide hyaluronique est présent dans la composition sous forme d'hyaluronate de sodium.

La composition ophtalmique selon l'invention étant une composition dépourvue de conservateurs, il est nécessaire de la traiter au cours de sa fabrication de manière à éviter et prévenir les contaminations par des pathogènes. Ces étapes peuvent avoir des effets négatifs sur la composition, sa turbidité voir sa stabilité dans le temps. Pour répondre à l'objectif de l'invention de préparer une composition hyperosmolaire d'acide hyaluronique, de préférence dépourvue d'agents conservateurs, il est avantageux d'employer de l'acide hyaluronique ayant une viscosité intrinsèque allant de 1,4 à 1,9 m³/kg.

La composition selon l'invention comprend préférentiellement de 0,01 à 0,025 % en poids d'acide hyaluronique, en particulier sous sa forme d'hyaluronate de sodium, avantageusement de 0,1 à 0,2 % en poids, en particulier environ 0,15 % en poids.

Selon un mode préférentiel de réalisation de l'invention, la composition comprend
- de 0,01 à 0,25% de hyaluronate de sodium, en particulier de 0,1 à 0,2 % en poids, plus particulièrement environ 0,15 % en poids,
- de 2 à 5 % de chlorure de sodium, en particulier de 4 à 5 % en poids, plus particulièrement 5 % en poids, et
- de l'eau.

Selon un mode particulier de réalisation de l'invention, la composition ophtalmique comprend
- de l'acide hyaluronique ou un sel physiologiquement acceptable de ce dernier
- un ou plusieurs agents d'osmolarité
- un tampon pour maintenir un pH compris entre 6 et 7, et
- de l'eau,
les constituants et leurs quantités étant avantageusement choisis par l'homme du métier pour obtenir une osmolarité allant de 1500 à 2000 mOsm/kg et une viscosité allant de 15 à 100 centipoises.

Selon un mode plus préférentiel de réalisation de l'invention, la composition ophtalmique est constituée par
- de 0,01 à 0,25% de hyaluronate de sodium, en particulier de 0,1 à 0,2 % en poids, plus particulièrement environ 0,15 % en poids,
- de 2 à 5 % de chlorure de sodium, en particulier de 4 à 5 % en poids, plus particulièrement 5 % en poids,
- de 0,05 à 0,5 % d'HPMC, particulièrement de 0,1 à 0,4 % en poids, plus particulièrement de 0,2 à 0,3 % en poids, encore plus particulièrement environ 0,25 % en poids
- un tampon pour un pH compris entre 6 et 7, et
- de l'eau.

L'invention concerne également un procédé de fabrication d'une composition ophtalmique telle que définie précédemment et dans les exemples. La composition ophtalmique selon l'invention combine plusieurs difficultés quant à sa préparation puisqu'il s'agit d'une solution, qui :
- contient de l'acide hyaluronique
- avec une forte concentration en agent d'osmolarité, et
- ne contient pas d'agents conservateurs.

Le procédé selon l'invention comprend avantageusement les étapes suivantes :
- Préparation d'une première solution aqueuse comprenant l'agent d'hyperosmolarité et le cas échéant un ou plusieurs agents de viscosité et/ou un ou plusieurs tampons (solution B),
- Préparation d'une deuxième solution aqueuse comprenant l'acide hyaluronique ou son sel acceptable (solution A),
- Transfert de la solution A dans la solution B,
- Le cas échéant ajustement du pH,
- Homogénéisation de la solution et le cas échéant ajustement final du pH, et
- Filtration stérilisante.

Les différentes étapes de préparation de solutions, homogénéisation et filtration stérilisante sont bien connues de l'homme du métier. La composition obtenue est de préférence conditionnée dans des conditionnements stériles, avantageusement dans des dispositifs appropriés pour sa conservation et son usage.

La composition selon l'invention est conditionnée dans un dispositif approprié permettant à la fois sa conservation à l'abri des contaminations par des pathogènes, et la libération d'une quantité appropriée de composition lors de son emploi pour son application sur l'œil, de préférence dans le sac conjonctival inférieur.

La libération se fait sous forme de gouttes. De préférence, le dispositif de conditionnement et de distribution de composition selon l'invention permettent de libérer des gouttes d'un poids compris entre 0.03 et 0.15 g.

L'invention concerne également la composition ophtalmique telle que définie précédemment et dans les exemples, pour son utilisation dans le traitement de l'œdème cornéen, plus particulièrement pour le traitement de l'œdème cornéen chronique (OCC).

Le traitement consiste généralement à instiller 1 à 2 gouttes de composition selon l'invention jusqu'à 4 fois par jour, en commençant de préférence par le matin, au réveil du sujet atteint d'œdème cornéen, particulièrement d'OCC.

L'intervalle entre les applications va de 15' et 3 heures, de préférence de 30 minutes environ, pouvant aller de 1 à 2 heures, en moyenne de 1 heure 30 minutes entre chaque application, plus préférentiellement environ 30'.

De manière avantageuse, le traitement comprend une première application dans l'œil du sujet atteint d'œdème cornéen, plus particulièrement d'OCC, d'une dose appropriée de composition suivie d'une deuxième application d'une dose appropriée.

La deuxième application peut être faite 1 à 2h suivant la première application. Elle peut également être faite de préférence 30' après la première application.

La dose appropriée correspond à 1 ou 2 gouttes de composition.

Le traitement comprend avantageusement une troisième application d'une dose appropriée, de préférence 30 minutes après la deuxième application, pouvant aller jusqu'à 1 à 2 heures suivant la deuxième application, optionnellement suivie d'une quatrième application d'une dose appropriée, de préférence 30 minutes après la troisième application, pouvant aller jusqu'à 1 à 3 heures suivant la troisième application.

Selon un mode particulier de réalisation de l'invention, le traitement comprend l'application dans l'œil du sujet atteint d'œdème cornéen, plus particulièrement d'OCC d'une dose appropriée de composition répétée au moins 3 fois par jour, de préférence 4 fois par jour, à un intervalle entre les applications entre 15' et 3 heures, de préférence de 30 minutes environ, pouvant aller de 1 à 2 heures, en moyenne de 1 heure 30 minutes entre chaque application, plus préférentiellement environ 30'.

De manière préférentielle, la première application se fait le matin au réveil du patient ou peu après.

L'invention concerne également une méthode de traitement de l'œdème cornéen, en particulier de l'œdème cornéen chronique, chez un sujet qui est atteint d'œdème cornéen, ladite méthode consistant à appliquer dans l'œil dudit sujet 1 ou plusieurs gouttes de composition selon l'invention, selon les schémas thérapeutiques décrits précédemment.

En particulier, la présente invention concerne un procédé de traitement de l'œdème cornéen chez un patient atteint d'œdème cornéen, le procédé comprenant l'application dans l'œil du sujet atteint d'œdème cornéen, plus particulièrement d'OCC, d'une dose appropriée de composition selon l'invention répétée au moins 3 fois par jour, de préférence 4 fois par jour.

L'intervalle entre les applications est de préférence de 30 minutes environ, pouvant aller de 1 à 2 heures, en moyenne de 1 heure 30 minutes entre chaque application.

Avantageusement, la première application se fait le matin au réveil du patient ou peu après.

L'application de la composition se fait par instillation de gouttes, de préférence dans le sac conjonctival inférieur de l'œil, le sujet regardant vers le haut et en tirant légèrement la paupière inférieure vers le bas. Après instillation, il est recommandé de garder l'œil fermé 1 à 2 minutes.

Pour un sujet atteint d'OCC, le traitement est avantageusement mis en œuvre pendant une durée d'au moins 2 semaines, allant jusqu'à 4 semaines ou plus. La composition selon l'invention est particulièrement appropriée pour un traitement de longue durée, c'est à dire pouvant aller jusqu'à plusieurs mois.

### EXEMPLES

### Exemple 1 - Composition

On prépare une composition ophtalmique comprenant les constituants suivants :

| Ingrédients | % | Rôle |
|---|---|---|
| Eau ppi | 92,14 | Excipent |
| Chlorure de sodium | 5,00 | Agent d'hyperosmolarité |
| Citrate tri-sodique dihydraté | 1,45 | Tampon |
| Acide citrique monohydraté (solution à 1%) | 1,01 | Tampon |
| HPMC | 0,25 | Agent épaississant |
| Hyaluronate de sodium | 0,15 | Agent lubrifiant |

Le pH est tamponné entre 6,2 et 6,8.

La composition est préparée selon la procédure suivante.

Dissolution des matières premières :
- Préparation de la solution B

On ajoute l'HPMC sous agitation à de l'eau purifiée chauffée. La solution est ensuite refroidie à température ambiante. On ajoute alors sous agitation le citrate trisodique dihydraté jusqu'à dissolution complète, puis le chlorure de sodium jusqu'à dissolution complète, puis l'acide citrique, jusqu'à dissolution complète.
- Préparation de la solution A

On ajoute lentement et sous agitation l'hyaluronate de sodium à de l'eau purifiée. L'agitation est maintenue plusieurs heures jusqu'à dissolution complète de l'hyaluronate de sodium.
- Transfert der la solution A dans la solution B
- Ajustement du pH entre 6,2 et 6,8
- Homogénéisation finale et ajustement final du pH
- Filtration stérilisante avec filtre stérilisant muni d'une cartouche hydrophile 0,22 µm
- Remplissage des récipients stériles.

La solution obtenue a une osmolarité de 1750 mOsm/kg (+/- 150). Elle est conservée dans les récipients stériles. Elle reste stable à température ambiante jusqu'à au moins 24 mois, et 1 mois après ouverture.

### Exemple 2 - Traitement de l'OCC

Un essai clinique portant sur 20 patients souffrant d'œdème cornéen avéré, lié à une insuffisance endothéliale, a été conduit sur une durée de 28 jours. Une à deux gouttes étaient instillées 4 fois par jour dans l'œil atteint, à 1h30 d'intervalle, en commençant la première instillation à 8h15. La moyenne d'âge des patients était de 72,2 ans.

L'évolution de l'acuité visuelle (mesurée avec échelle EDTRS) et de l'épaisseur cornéenne (mesurée par pachymétrie ultrasonique) a été évaluée en comparant les valeurs entre la visite d'inclusion (sept jours avant le début du traitement) et les visites de suivi (sept et vingt-huit jours après le 1^{er} jour de traitement).

Dès le 7^{ème} jour de traitement, l'acuité visuelle s'améliore significativement de 5 lettres en moyenne (p<0,001 test de Wilcoxon apparié). L'épaisseur cornéenne a également diminué significativement (p=0.033 test de Wilcoxon apparié). L'amélioration fonctionnelle est retrouvée à 28 jours d'instillation. Aucun effet indésirable n'a été relevé au cours de l'étude. Après 28 jours de traitement, la solution hyperosmolaire sans conservateur et contenant du hyaluronate de sodium a amélioré significativement l'acuité visuelle des patients et a été très bien tolérée.

## Revendications

1. Composition ophtalmique hyperosmolaire **caractérisée en ce qu'**elle comprend de l'acide hyaluronique ou un sel acceptable de ce dernier et un agent d'hyperosmolarité et **en ce qu'**elle présente une osmolarité allant de plus de 1500 à 2000 mOsm/l et une viscosité allant de 15 à 100 mPa.s (15 à 100 centipoises), mesurée selon les préconisations de la pharmacopée européenne 2.2.10 avec un viscosimètre rotatif à 25°C et 100 s⁻¹.

2. Composition selon la revendication 1, **caractérisée en ce que** l'agent d'hyperosmolarité est choisi parmi le chlorure de sodium, le chlorure de potassium et le glycérol.

3. Composition selon la revendication 2, **caractérisée en ce qu'**elle comprend 5 % en poids de chlorure de sodium.

4. Composition selon l'une des revendications 1 à 3, **caractérisée en ce qu'**elle comprend de 0,01 à 0,25 % en poids d'hyaluronate de sodium.

5. Composition selon l'une des revendications 1 à 4, **caractérisée en ce qu'**elle comprend un agent de viscosité choisi parmi hydroxypropylméthylcellulose, hydroxyethylcellulose, carboxymethylcellulose, carbomères, gels agar, polyvinylpyrrolidone et alcool polyvinylique.

6. Composition selon la revendication 5, **caractérisée en ce qu'**elle comprend de 0,05 à 0,5 % en poids d'hydroxypropylmethyl cellulose.

7. Composition selon la revendication 1, **caractérisée en ce qu'**elle est constituée par
- de 0,01 à 0,25% de hyaluronate de sodium,
- 5 % de chlorure de sodium,
- de 0,05 à 0,5 % d'HPMC,
- d'un tampon pour un pH compris entre 6 et 7, et
- de l'eau.

8. Composition ophtalmique selon l'une des revendications 1 à 7, pour son utilisation dans le traitement de l'œdème cornéen.

9. Composition pour son utilisation selon la revendication 8, **caractérisée** en l'œdème cornéen est un œdème cornéen chronique (OCC).

10. Composition pour son utilisation selon l'une des revendications 8 ou 9, **caractérisée en ce que** le traitement comprend une première application dans l'œil du sujet atteint d'œdème cornéen, plus particulièrement d'OCC, d'une dose appropriée de composition suivie d'une deuxième application d'une dose appropriée 30 minutes à 1 heure 30 suivant la première application.

11. Composition pour son utilisation selon la revendication 10, **caractérisée en ce qu'**elle comprend une troisième application d'une dose appropriée 30 minutes à 1 heure 30 suivant la deuxième application.

12. Composition pour son utilisation selon l'une des revendications 8 à 11, **caractérisée en ce que** le traitement comprend l'application dans l'œil du sujet atteint d'œdème cornéen, plus particulièrement d'OCC, d'une dose appropriée de composition répétée au moins 3 fois par jour, de préférence 4 fois par jour, à un intervalle entre les applications de 30 minutes.

13. Composition pour son utilisation selon l'une des revendications 8 à 12, **caractérisée en ce que** la première application se fait le matin au réveil du patient ou peu après.

14. Composition pour son utilisation selon l'une des revendications 9 à 13, **caractérisée en ce que** le traitement est mis en œuvre pendant une durée d'au moins 2 semaines, pouvant aller jusqu'à au moins 4 semaines.

## Patentansprüche

1. Hyperosmolare ophthalmische Zusammensetzung, **dadurch gekennzeichnet, dass** sie Hyaluronsäure oder ein annehmbares Salz davon und ein Hyperosmolaritätsmittel umfasst, und dadurch, dass sie eine Osmolarität von über 1500 bis 2000 mOsm/l und eine Viskosität von 15 bis 100 mPa.s (15 bis 100 Centipoise) aufweist, die gemäß den Empfehlungen des Europäischen Arzneibuchs 2.2.10 mit einem Rotationsviskosimeter bei 25°C und 100 s⁻¹ gemessen wurde.

2. Zusammensetzung nach Anspruch 1, **dadurch gekennzeichnet, dass** das Hyperosmolaritätsmittel unter Natriumchlorid, Kaliumchlorid und Glycerin ausgewählt ist.

3. Zusammensetzung nach Anspruch 2, **dadurch gekennzeichnet, dass** sie 5 Gew.-% Natriumchlorid umfasst.

4. Zusammensetzung nach einem der Ansprüche 1 bis 3, **dadurch gekennzeichnet, dass** sie von 0,01 bis 0,25 Gew.-% Natriumhyaluronat umfasst.

5. Zusammensetzung nach einem der Ansprüche 1 bis 4, **dadurch gekennzeichnet, dass** sie ein Viskositätsmittel umfasst, das unter Hydroxypropylmethylcellulose, Hydroxyethylcellulose, Carboxymethylcellulose, Carbomeren, Agar-Gelen, Polyvinylpyrrolidon und Polyvinylalkohol ausgewählt ist.

6. Zusammensetzung nach Anspruch 5, **dadurch gekennzeichnet, dass** sie von 0,05 bis 0,5 Gew.-% Hydroxypropylmethylcellulose umfasst.

7. Zusammensetzung nach Anspruch 1, **dadurch gekennzeichnet, dass** sie besteht aus
- von 0,01 bis 0,25% Natriumhyaluronat,
- von 5% Natriumchlorid,
- von 0,05 bis 0,5 HPMC,
- einem Puffer für einen pH-Wert zwischen 6 und 7, und
- Wasser.

8. Ophthalmische Zusammensetzung nach einem der Ansprüche 1 bis 7 zur Verwendung bei der Behandlung eines Hornhautödems.

9. Zusammensetzung zur Verwendung nach Anspruch 8, **dadurch gekennzeichnet, dass** das Hornhautödem ein chronisches Hornhautödem ist.

10. Zusammensetzung zur Verwendung nach einem der Ansprüche 8 oder 9, **dadurch gekennzeichnet, dass** die Behandlung eine erste Anwendung einer geeigneten Dosis an Zusammensetzung in dem Auge der an dem Hornhautödem, insbesondere dem chronischen Hornhautödem, erkrankten Person, gefolgt von einer zweiten Anwendung einer geeigneten Dosis 30 Minuten bis 1 Stunde 30 Minuten nach der ersten Anwendung umfasst.

11. Zusammensetzung zur Verwendung nach Anspruch 10, **dadurch gekennzeichnet, dass** sie eine dritte Anwendung einer geeigneten Dosis 30 Minuten bis 1 Stunde 30 Minuten nach der zweiten Anwendung umfasst.

12. Zusammensetzung zur Verwendung nach einem der Ansprüche 8 bis 11, **dadurch gekennzeichnet, dass** die Behandlung die Anwendung einer geeigneten Dosis einer wiederholten Zusammensetzung in dem Auge der an dem Hornhautödem, insbesondere dem chronischen Hornhautödem, erkrankten Person mindestens 3 Mal am Tag, vorzugsweise 4 Mal am Tag, mit einem zeitlichen Abstand von 30 Minuten zwischen den Anwendungen umfasst.

13. Zusammensetzung zur Verwendung nach einem der Ansprüche 8 bis 12, **dadurch gekennzeichnet, dass** die erste Anwendung am Morgen beim Erwachen des Patienten oder kurz danach erfolgt.

14. Zusammensetzung zur Verwendung nach einem der Ansprüche 9 bis 13, **dadurch gekennzeichnet, dass** die Behandlung während einer Dauer von mindestens 2 Wochen durchgeführt wird und bis zu mindestens 4 Wochen dauern kann.

## Claims

1. A hyperosmolar ophthalmic composition **characterized in that** it comprises hyaluronic acid or an acceptable salt thereof and a hyperosmolar agent and **in that** it has an osmolarity ranging from more than 1500 to 2000 mOsm/L and a viscosity ranging from 15 to 100 mPa.s (15 to 100 centipoise), measured according to the recommendations of European Pharmacopoeia 2.2.10 with a rotating viscometer at 25°C and 100 s⁻¹.

2. The composition according to claim 1, **characterized in that** the hyperosmolarity agent is selected from sodium chloride, potassium chloride and glycerol.

3. The composition according to claim 2, **characterized in that** it comprises 5 wt% sodium chloride.

4. The composition according to one of claims 1 to 3, **characterized in that** it comprises from 0.01 to 0.25 wt% sodium hyaluronate.

5. The composition according to one of claims 1 to 4, **characterized in that** it comprises a viscosity agent selected from hydroxypropylmethylcellulose, hydroxyethylcellulose, carboxymethylcellulose, carbomers, agar gels, polyvinylpyrrolidone and polyvinyl alcohol.

6. The composition according to claim 5, **characterized in that** it comprises from 0.05 to 0.5 wt% hydroxypropylmethyl cellulose.

7. The composition according to claim 1, **characterized in that** it consists of
- from 0.01 to 0.25% sodium hyaluronate,
- from 5% sodium chloride,
- from 0.05 to 0.5% HPMC,
- a buffer for a pH between 6 and 7, and
- water.

8. The ophthalmic composition according to one of claims 1 to 7, for use in the treatment of corneal edema.

9. The composition for use according to claim 8, **characterized in that** the corneal edema is a chronic corneal edema (CCE).

10. The composition for use according to one of claims 8 or 9, **characterized in that** the treatment comprises a first application in the eye of the subject suffering from corneal edema, more particularly CCE, of an appropriate dose of the composition followed by a second application of an appropriate dose 30 minutes to 1.5 hours after the first application.

11. The composition for use according to claim 10, **characterized in that** it comprises a third application of an appropriate dose 30 minutes to 1.5 hours following the second application.

12. The composition for use according to one of claims 8 to 11, **characterized in that** the treatment comprises the application in the eye of the subject suffering from corneal edema, more particularly CCE, of an appropriate dose of the composition repeated at least 3 times a day, preferably 4 times a day, at an interval of 30 minutes between applications.

13. The composition for use according to one of claims 8 to 12, **characterized in that** the first application is made in the morning when the patient wakes up or shortly thereafter.

14. The composition for use according to one of claims 9 to 13, **characterized in that** the treatment is carried out for a period of at least 2 weeks up to at least 4 weeks.
